(19) Europäisches Patentamt
European Patent Office
Office européen des brevets

(11) Publication number: **0 041 826 B1**

(12) **EUROPEAN PATENT SPECIFICATION**

(45) Date of publication of patent specification: **28.12.83**

(51) Int. Cl.³: **C 07 C 119/06, A 61 K 31/195**

(21) Application number: **81302466.8**

(22) Date of filing: **03.06.81**

(54) **4-(N-(substituted-benzylidene)aminomethyl)cyclohexane-1-carboxylic acids.**

(30) Priority: **04.06.80 JP 75055/80**
**09.01.81 JP 1790/81**

(43) Date of publication of application:
**16.12.81 Bulletin 81/50**

(45) Publication of the grant of the patent:
**28.12.83 Bulletin 83/52**

(84) Designated Contracting States:
**BE CH DE FR GB IT LI NL SE**

(56) References cited:
**CHEMICAL ABSTRACTS, vol. 69, no. 19, 4th November 1968, page 7187, column 2, no. 76971n, Columbus, Ohio, U.S.A.**

(73) Proprietor: **KUREHA KAGAKU KOGYO KABUSHIKI KAISHA**
**9-11 Horidome-cho 1-chome Nihonbashi Chuo-ku**
**Tokyo (JP)**

(72) Inventor: **Takita, Hitoshi**
**1-8 Toyotama-kita**
**Nerima-ku Tokyo (JP)**
Inventor: **Mukaida, Yutaka**
**No. 21 Daiichi-Kurehasoh, 3-10-13 Nerima**
**Nerima-ku Tokyo (JP)**
Inventor: **Noda, Sakuo**
**No. 103 Kureha Ohkubo-Shataku 3-26-1**
**Hyakunin-cho**
**Shinjuku-ku Tokyo (JP)**
Inventor: **Kobayashi, Hidetoshi**
**2-21-2 Zenpukuji**
**Suginami-ku Tokyo (JP)**

(74) Representative: **Myerscough, Philip Boyd et al,**
**J.A.Kemp & Co. 14, South Square Gray's Inn**
**London, WC1R 5EU (GB)**

**EP 0 041 826 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

0 041 826

4-[N-(substituted-benzylidene)aminomethyl]cyclohexane-1-carboxylic acids

This invention relates to 4-[N-(substituted-benzylidene)aminomethyl]cyclohexane-1-carboxylic acids, to their production and to pharmaceutical compositions containing them.

The present invention provides a 4-[N-(substituted-benzylidene-aminomethyl]cyclohexane-1-carboxylic acid (hereinafter referred to as the compound of the invention) represented by the general formula (I):

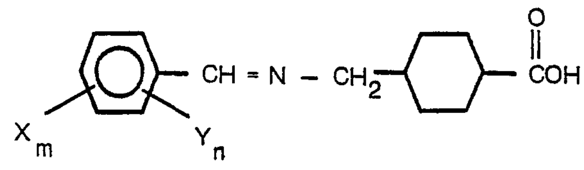

(I)

wherein X is hydroxyl, Y is methoxy, and $m$ is 0 or an integer from 1 to 5 and $n$ is 0 or an integer from 1 to 5 such that $1 \leqslant m + n \leqslant 5$, or a salt or an ester thereof, with the proviso that when $n$ is 0, $m$ is 2 and one of the two hydroxyls is in the para-position of the benzene nucleus, then the other hydroxyl is not in the meta-position of the benzene nucleus. The proviso therefore excludes 4-[N-(3′,4′-dihydroxybenzylidene)aminomethyl]-cyclohexane-1-carboxylic acid and its salts and esters, which compounds are the subject of the European patent application 81302467.6 (publication number 41.827) of the same effective date. In the general formula (I), the cyclohexane ring may be in either the trans- or the cis form.

The salts of the compound of the invention include alkali or alkaline earth metal salts such as the sodium salt, potassium salt, calcium salt or magnesium salt, ammonium salts or primary-, secondary-, tertiary- or quaternary ammonium salts. The esters are preferably lower alkyl esters in which the alkyl group has 1 to 3 carbon atoms, such as methyl-, ethyl- or n- or isopropyl.

The compounds of the invention and their salts and esters are preferably prepared by the process described below, although it may be prepared by the other methods. Preferably, therefore, compounds of the general formula (I) and salts and esters thereof are prepared by reacting a substituted-benzaldehyde represented by the general formula (II):

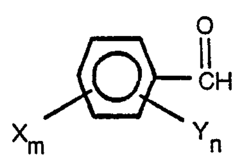

(II)

wherein X, Y, $m$ and $n$ are as defined above, with 4-amino-methyl-cyclohexane-1-carboxylic acid which has the formula (III):

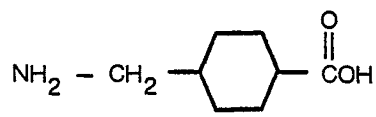

(III)

wherein the cyclohexane ring can be in either the trans-or cis form, or an ester, preferably a lower alkyl ester, thereof. A dehydration-condensation reaction occurs. The resulting compound of the general formula (I) can optionally be converted to a salt or ester thereof. Alternatively, if the resulting compound is an ester of a compound of the general formula (I), this ester can optionally be converted into a compound of the general formula (I) or a salt or other ester thereof.

The reaction between the compound of formula (II) and the compound of the formula (III) or an ester thereof is preferably carried out in an organic solvent at a temperature lower than 150°C, preferably 0 to 120°C under an atmosphere of an inert gas. At temperatures higher than 150°C, the yield of the desired product is reduced because of various side reactions. Any organic solvent may be used for the reaction provided it does not participate in the reaction. For example, a lower alcohol such as methanol or ethanol, benzene, toluene, dimethylformamide, or acetonitrile is conventionally used for the reaction.

Since the reaction takes place with dehydration, the reaction is preferably carried out in the presence of a dehydrating agent or while removing water formed by the reaction by refluxing the solvent. An anhydrous form of a lower alcohol, such as an anhydrous methanol or ethanol, can be used as the solvent and at the same time acts as the dehydrating agent. The compound of the invention (acid- or ester form) can be isolated by treating the reaction mixture in a known manner after the reaction has been completed.

A salt of the compound of the invention can be prepared by the conventional method involving

neutralization by using a base such as a hydroxide, carbonate or bicarbonate of an alkali or alkaline earth metal, for example sodium, potassium, calcium or magnesium, or of ammonia or a primary-, secondary- or tertiary amine or quaternary ammonium salt. For example, a sodium salt can be obtained by neutralizing a compound of the invention (an acid form) with an alcoholic or aqueous solution of sodium hydroxide under an atmosphere of an inert gas at a temperature lower than 100°C, usually at from 0 to 50°C.

The compound of the invention shows an inhibitory effect on platelet aggregation and/or polynuclear leukocyte migration, and a low acute toxicity, as will be shown in Example 10. Accordingly, the compound of the invention is useful in therapy for the treatment of, for example, inflammation, thrombosis, encephalorrhagia, hypertension, asthma or cancer, and especially for treating chronic diseases such as rheumatism or systemic lupus erythematosus (SLE). The present invention therefore also provides a pharmaceutical composition comprising a compound of the general formula (I) or a pharmaceutically acceptable salt or ester thereof as active ingredient, together with a pharmaceutically acceptable carrier and/or adjuvant.

The composition of the invention can be administered perorally, rectally or by injection in the various dosage forms. Two or more of the compounds of the general formula (I) and their pharmaceutically acceptable salts or esters may be used. Other pharmaceutically active materials may be incorporated in the pharmaceutical composition.

The dosage form of the composition may be as a tablet, sublingual tablet, powder, capsule, troch, aqueous or oily solution, suspension, emulsion, syrup or aqueous or oily injection. Examples of the carrier include water, gelatin, lactose, starch, pectin, magnesium stearate, stearic acid, talc, vegetable oil, gum arabic, polyalkylene glycol, vaseline, sorbitan tri-oleate, polyoxyethylene-sorbitan mono-oleate, alkylphenol, aliphatic alcohols and polyvinyl pyrrolidone. In the composition, if necessary, an edulcorant, flavour, tinctorial agent, preservative, salt for osmoregulation or buffer, that is a conventional pharmaceutical adjuvant, may be used.

The content of the compound of the invention or pharmaceutically acceptable salt or ester thereof in a pharmaceutical composition of the invention may be suitably varied, for example, within a range of 0.01%—100% by weight of the composition, preferably 0.05%—80% by weight of the composition.

The pharmaceutical composition of the invention can be administered to a human or animal parentally, for example, rectally, by injection (hypodermic, intramuscular or intravenous, or drip) or preferably perorally (for example sublingually). A dose of the pharmaceutical composition of the invention can consist of $10^{-7}$ to $5 \times 10^{-4}$ kg (0.1 to 500 mg), preferably $5 \times 10^{-7}$ to $2 \times 10^{-4}$ kg (0.5 to 200 mg) per day per kilogram of body weight in the case of peroral administration to a human, and $10^{-8}$ to $2 \times 10^{-4}$ kg (0.01 to 200 mg), preferably $10^{-7}$ to $10^{-4}$ kg (0.1 to 100 mg) in the case of parenteral administration. The pharmaceutical composition can be administered one to four times a day. However the dose of the pharmceutical composition depends on for example age, the individual being treated and the condition of a disease. Doses outside the above-mentioned range may be used.

The following Examples illustrate the present invention.

## Example 1

Preparation of trans - 4 - [N - (2′ - hydroxybenzylidene)amino - methyl[cyclohexane - 1 - carboxylic acid:

$1.550 \times 10^{-3}$ kg (1.550 g) (12.7 mmol) of 2-hydroxybenzaldehyde was dissolved in $3 \times 10^{-5} m^3$ (30 ml) of dehydrated and purified methanol. The resulting solution was added dropwise to $1.994 \times 10^{-3}$ kg (1.994 g) (12.7 mmol) of trans - 4 - aminomethyl - cyclohexane - 1 - carboxylic acid under an atmosphere of nitrogen. The resulting mixed solution was refluxed while stirring for 3 hours. After cooling the reaction mixture to room temperature, the yellow crystals that deposited were filtered off, washed with methanol and vacuum-dried. $2.62 \times 10^{-3}$ kg (2.62 g) of trans - 4 - [N - (2′ - hydroxybenzylidene) - aminomethyl]cyclohexane - 1 - carboxylic acid was obtained in a yield of 83.6%.

The characteristics of this compound were as follows:

(1) fusing point; 151—153°C

(2) elementary analysis;

| | C(%) | H(%) | N(%) |
|---|---|---|---|
| theoretical: | 68.94 | 7.33 | 5.36 |
| experimental: | 69.1 | 7.2 | 5.4 |

(3) nuclear magnetic resonance (NMR) spectrum in dimethylsulphoxide;
$\delta = 0.95$—2.0 (9H, m), 2.0—2.15 (1H), 3.45 (2H, d), 6.65 (1H), 6.94 (1H), 7.30 (1H), 7.42 (1H), 8.50 (1H), 13.0 (1H).

Example 2

Preparation of sodium trans - 4 - [N - (2′ - hydroxybenzylidene)aminomethyl]cyclohexane - 1 - carboxylate:

1.305 × $10^{-3}$ kg (1.305 g) (5 mmol) of trans - 4 - [N - (2′ - hydroxybenzylidene)aminomethyl]cyclohexane - 1 - carboxylic acid obtained in Example 1 was added under an atmosphere of nitrogen to 5 × $10^{-5}m^3$ (50 ml) (5 mmol) of 0.1 N aqueous solution of sodium hydroxide. The resulting mixture was stirred for 2 hours at room temperature to obtain a yellow solution. The yellow solution was filtered and then freeze-dried. 1.42 × $10^{-3}$ kg (1.42 g) of sodium trans - [N - - 2′ - hydroxybenzylidene)aminomethyl]cyclohexane - 1 - carboxylate was obtained.

The characteristics of this sodium salt were as follows:

(1) melting point; higher than 230°C,

(2) elementary analysis:

| | C(%) | H(%) | N(%) |
|---|---|---|---|
| theoretical: | 64.63 | 6.78 | 4.71 |
| experimental: | 64.0 | 6.9 | 4.9 |

(3) Infrared (IR) absorption spectrum:

$\upsilon(cm^{-1}) = 3400, 2920, 1633, 1620, 1580, 1540, 1500, 1420, 1290, 1048, 858, 752.$

Example 3

Preparation of trans - 4 - [N - (4′ - hydroxybenzylidene)aminomethyl]cyclohexane - 1 - carboxylic acid:

1.550 × $10^{-3}$ kg (1.550 g) 12.7 mmol) of 4-hydroxybenzaldehyde was reacted with 1.994 × $10^{-3}$ kg (1.994 g) (12.7 mmol) of trans - 4 - aminomethyl - cyclohexane - 1 - carboxylic acid while refluxing for 5 hours according to the method of Example 1. 1.26 × $10^{-3}$ kg (1.26 g) of trans - 4 - [N - (4′ - hydroxybenzylidene)aminomethyl]cyclohexane - 1 - carboxylic acid was obtained in the form of orange crystals in a yield of 38.0%.

The characteristics of this compound were as follows:

(1) melting point; 208°C with decomposition,

(2) elementary analysis;

| | C(%) | H(%) | N(%) |
|---|---|---|---|
| theoretical: | 68.94 | 7.33 | 5.36 |
| experimental: | 69.0 | 7.1 | 5.50 |

(3) NMR spectrum;

$\delta$ × 0.9—2.0 (9H, m), 2.1—2.2 (1H), 3.35 (2H, d), 6.80 (2H, d), 7.55 (2H, d), 8.12 (1H, s).

Example 4

Preparation of trans - 4 - [N - (2′,3′ - dihydroxybenzylidene)aminomethyl]cyclohexane - 1 - carboxylic acid and sodium salt thereof:

1.753 × $10^{-3}$ kg (1.753 g) (12.7 mmol) of 2,3-dihdyroxybenzaldehyde was reacted with 1.994 × $10^{-3}$ kg (1.994 g) (12.7 mmol) of trans - 4 - aminomethyl - cyclohexane - 1 - carboxylic acid according to the method of Example 1. 2.53 × 10 kg (2.53 g) of trans - 4 - 4[N - (2′,3′ - dihydroxybenzylidene)aminomethyl]cyclohexane - 1 - carboxylic acid was obtained in the form of yellow crystals in a yield of 71.9%.

The characteristics of this compound were as follows:

(1) melting point; 190—192°C,

(2) elementary analysis;

| | C(%) | H(%) | N(%) |
|---|---|---|---|
| theoretical: | 64.97 | 6.91 | 5.05 |
| experimental: | 64.6 | 6.9 | 4.9 |

(3) NMR spectrum;

$\delta$ = 0.97—2.00 (9H, m), 2.15 (1H), 2.65 (2H, d), 4.04 (2H), 6.53—6.88 (3H), 8.45 (1H).

Furthermore, this compound of the invention was neutralized by an equivalent amount of sodium hydroxide to obtain sodium trans - 4 - [N - (2′,4′ - dihydroxybenzylidene)aminomethyl]cyclohexane - 1 - carboxylate.

Example 5

Preparation of trans - 4 - [N - (2′,4′ - dihydroxybenzylidene)aminomethyl]cyclohexane - 1 - carboxylic acid:

1.753 × $10^{-3}$ kg (1.753 g) (12.7 mmol) of 2.4-dihydroxybenzaldehyde was reacted with

1.994 x $10^{-3}$ kg (1.994 g) (12.7 mmol) of trans - 4 - aminomethylcyclohexane - 1 - carboxylic acid according to the method of Example 1. 3.02 x $10^{-3}$ kg (3.02 g) of trans - 4 - [N - (2',4' - dihydroxybenzylidene)aminomethyl - cyclohexane - 1 - carboxylic acid was obtained in the form of orange-yellow crystals in a yield of 85.8%.

The characteristics of this compound were as follows:

(1) melting point; 225°C with decomposition,

(2) elementary analysis;

| | C(%) | H(%) | N(%) |
|---|---|---|---|
| theoretical: | 64.97 | 6.91 | 5.05 |
| experimental: | 64.5 | 7.1 | 5.3 |

(3) NMR spectrum;

$\delta$ = 1.06—1.85 (9H, m), 2.01 (1H), 2.52 (2H), 3.39 (2H), 6.17 (1H), 6.28 (1H), 7.17 (1H), 8.29 (1H), 10—12 (1H).

Example 6

Preparation of trans - 4 - [N - (2' - methoxybenzylidene)aminomethyl]cyclohexane - 1 - carboxylic acid and sodium salt thereof:

1.727 x 10 kg (1.727 g) (12.7 mmol) of 2-methoxybenzaldehyde was reacted with 1.994 x $10^{-3}$ kg (1.994 g) (12.7 mmol) of trans - 4 - aminomethylcyclohexane - 1 - carboxylic acid according to the method of Example 1. 2.21 x $10^{-3}$ kg (2.21 g) of trans - 4 - [N - (2' - methoxybenzylidene)aminomethyl]cyclohexane 1 carboxylic acid in the form of colourless crystals was obtained in a yield of 63.3%.

The characteristics of this compound were as follows:

(1) melting point;174—175°C,

(2) elementary analysis;

| | C(%) | H(%) | N(%) |
|---|---|---|---|
| theoretical: | 69.79 | 7.69 | 5.09 |
| experimental: | 69.7 | 7.9 | 5.2 |

(3) NMR spectrum;

$\delta$ = 0.90—1.98 (9H, m), 2.10 (1H), 3.49 (2H, d), 3.83 (3H, s), 6.99 (1H), 7.22 (1H), 7.46 (1H), 7.83 (1H), 8.58 (1H).

The sodium salt of this compound was prepared by neutralization with an equivalent amount of sodium hydroxide, and sintering at about 170°C.

Example 7

Preparation of trans - 4 - [N - (3',4' - dimethoxybenzylidene)aminomethyl]cyclohexane - 1 - carboxylic acid:

2.12 x $10^{-3}$ kg (2.12 g) (12.72 mmol) of 3,4-dimethoxybenzaldehyde was reacted with 2.00 x $10^{-3}$ kg (2.00 g) (12.72 mmol) of trans - 4 - aminomethylcyclohexane - 1 - carboxylic acid in methanol according to the method of Example 1. 2.45 x $10^{-3}$ kg (2.45 g) of trans - 4 - [N - (3',4' - dimethoxybenzylidene)aminomethyl]cyclohexane - 1 - carboxylic acid was obtained in the form of colourless flake-crystals in a yield of 63.1%.

The characteristics of this compound were as follows:

(1) melting point; 196—199°C,

(2) elementary analysis;

| | C(%) | H(%) | N(%) |
|---|---|---|---|
| theoretical: | 66.86 | 7.59 | 4.59 |
| experimental: | 66.6 | 7.5 | 4.6 |

(3) NMR spectrum;

$\delta$ = 1.02—1.83 (9H, m), 1.90—2.18 (1H), 3.37 (2H, d), 3.79 (6H, s), 6.99 (1H, d), 7.24 (1H, d), 7.34 (1H, s), 8.17 (1H, s).

Example 8

Preparation of trans - 4 - [N - (3',4',5' - trimethoxybenzylidene)aminomethyl]cyclohexane - 1 - carboxylic acid:

2.49 x $10^{-3}$ kg (2.49 g) (12.72 mmol) 3,4,5-trimethoxybenzaldehyde was reacted with 2.00 x $10^{-3}$ kg (2.00 g) (12.72 mmol) of trans - 4 - aminomethylcyclohexane - 1 - carboxylic acid in 6 x $10^{-5}$m³ (60 ml) of methanol while refluxing for 6.5 hours, according to the method of Example 1. 3.32 x $10^{-3}$ kg (3.32 g) of trans - 4 - [N - (3',4',5' - trimethoxybenzylidene)aminomethyl]cyclohexane - 1 - carboxylic acid was obtained in the form of colourless needle-crystals in a yield of 77.8%.

The characteristics of this compound were as follows:

(1) melting point;176—180°C,

(2) elementary analysis;

| | C(%) | H(%) | N(%) |
|---|---|---|---|
| theoretical: | 64.46 | 7.51 | 4.18 |
| experimental: | 64.3 | 7.5 | 4.1 |

(3) NMR spectrum;

$\delta$ = 1.00—190 (9H, m), 2.00—2.15 (1H), 3.40 (2H, d), 3.70 (3H, s), 3.81 (6H, s), 7.06 (2H, s), 8.18 (1H, s).

Example 9

Preparation of trans - 4 - [N - (3′ - methoxy - 4′ - hydroxybenzylidene)aminomethyl]cyclohexane - 1-carboxylic acid:

12.7 mmol of 3-methoxy-4-hydroxybenzaldehyde was reacted with 12.7 mmol of trans-4-amino-methyl-cyclohexane-1-carboxylic acid in 6 × $10^{-5}$ $m^3$ (60 ml) of methanol for 10 hours according to the method of Example 1. The reaction solution was filtered and methanol was evaporated off at 110 to 120°C under reduced pressure for 5 hours. A yellow powder of trans - 4 - [N - (3′ - methoxy - 4′-hydroxybenzylidene)aminomethyl]cyclohexane - 1 - carboxylic acid was obtained in a yield of 80.2%.

The characteristics of this compound were as follows:

(1) melting point; 205—206°C with decomposition.

(2) elementary analysis;

| | C(%) | H(%) | N(%) |
|---|---|---|---|
| theoretical: | 65.96 | 7.27 | 4.81 |
| experimental: | 65.4 | 7.3 | 4.5 |

(3) IR absorption spectrum; as shown in the accompanying drawing.

Example 10

Examination of pharmacological activity and acute toxicity:

The pharmacological activity and acute toxicity of the compounds of the invention and their salts and esters were examined. The specimens examined are as follows:

Specimen I; Sodium trans - 4 - [N - (2′ - hydroxybenzylidene)aminomethyl]cyclohexane - 1 - carboxylate obtained in Example 2,

II; Sodium trans - 4 - [N - (2′,3′ - dihydroxybenzylidene)aminomethyl]cyclohexane - 1 - carboxylate obtained in Example 4.

III; Sodium salt of trans - 45 - [N - (2′,4′ - dihydroxybenzylidene)aminomethyl]cyclohexane-1 - carboxylic acid obtained in Example 5, and

IV; Sodium trans - 4 - [N - (2′ - methoxybenzylidene)aminomethyl]cyclohexane - 1 - carboxylate obtained in Example 6.

Comparative Specimen V; indomethacin, and

VI; homochlorcyclizine.

Comparative Specimens V and VI having similar pharmacological activity of the compounds of the invention are commercially available in Japan.

The examination was carried out according to the methods described below:

(1) Examination for inhibitory effect on platelet aggregation:

Rabbit platelet-rich plasma (PRP) was used for Aggregation Tests. PRP was prepared by centrifugation of blood collected from the ear vein of a rabbit and diluted to a number-concentration of 300,000/$10^{-9}m^3$ (300,000/$\mu$l) with platelet-poor plasma. Platelet aggregation was induced by sodium arachidonate and monitored (measured) with a four-channel platelet aggregation Tracer PAT—4A (Niko Bioscience Co., Japan). An Aggregation Tracer tube containing 2.30 × $10^{-7}$ $m^3$ (230 $\mu$l) PRP was preincubated at 37°C for 5 minutes with each Specimen.

(2) Examination for inhibitory effect on polynuclear leukocyte migration:

Rat's polynuclear leukocyte migration was examined by the Boyden method (refer to Japanese J. Clin. Med., *27*(9), 172, (1969)) as follows. A mixture of 10 parts by volume of a culture filtrate of *E. coli* and one part by volume of serum was incubated at 37°C for one hour and diluted to 5 times its volume with a physiological salt solution. The mixture obtained was used as an attractant. After preincubating a polynuclear leukocyte suspension with each specimen for 10 minutes, the migration of polynuclear leukocytes was examined.

(3) Examination for acute toxicity:

Acute toxicity was examined by administering perorally an aqueous solution of each Specimen to female JCL—ICR mice 5 to 6 weeks after their birth.

The results are shown in the Table.

TABLE

| Specimen | Platelet aggregation $ID_{100}$ (μM)1) | Inhibition rate of polynuclear leukocyte migration (%)2) | | | | Acute toxicity $LD_{50}$ : $10^{-6}$ kg/kg (mg/kg)3) |
|---|---|---|---|---|---|---|
| | | 1μM | 10μM | 100μM | 1000μM | |
| $I_o$ | >1000 | 0 | 0 | 39 | 93 | ~2000 |
| II | 120 ~ 60 | 0 | 25 | 66 | 100 | >3000 |
| III | >1000 | 0 | 12 | 42 | 92 | >3000 |
| IV | 400 ~ 500 | 0 | 0 | 27 | 94 | >3000 |
| V | 0.5 ~ 1 | 0 | 0 | 25 | 79 | 30 |
| VI | >1000 | 15 | 48 | 80 | 100 | 350 |

Notes: 1) The minimum concentration (μM) of each Specimen for completely inhibiting (100%) the platelet aggregating effect by sodium arachidonate (tested at 400 μM).

2) The average value of the inhibition rate (%) of polynuclear leukocyte migration at each concentration (μM) of each Specimen.

3) Median lethal dose ($LD_{50}$), that is the dose for killing 50% of the mice, in unit of $10^{-6}$ kg (mg) per kilogram of body weight of a mouse.

As shown in the Table, the compounds of the invention (sodium salts) have an inhibitory effect on platelet aggregation and polynuclear leukocyte migration, and a low acute toxicity. Accordingly, the compounds of the invention can be used as an anti-inflammatory agent and for treating various other diseases.

Furthermore, it is confirmed that the sodium salt of the trans - 4 - [N - (3′,4′ - dimethoxybenzylidene) - aminomethyl]cyclohexane - 1 - carboxylic acid obtained in Example 7, of the trans - 4 - [N-(3′,4′,5′, - trimethoxybenzyl - idene)amino - methyl]cyclo - hexane - 1 - carboxylic acid obtained in Example 8 and of the trans - 4 - [N - (3′ - methoxy - 4′ - hydroxybenzylidene) - aminomethyl]cyclohexane - 1 - carboxylic acid obtained in Example 9 shows the similar pharmacological activity and low acute toxicity.

**Claims**

1. A compound represented by the general formula (I):

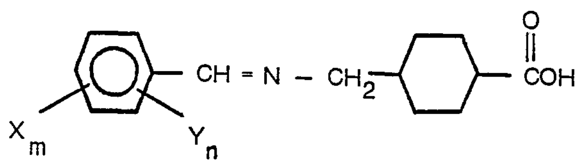

(I)

wherein X is hydroxyl, Y is methoxy, and *m* is 0 or an integer from 1 to 5 and *n* is 0 or an integer from 1 to 5 such that $1 \leqslant m + n \leqslant 5$, or a salt or an ester thereof, with the proviso that when *n* is 0, *m* is 2 and one of the two hydroxyls is in the para-position of the benzene nucleus, then the other hydroxyl is not in the meta-position of the benzene nucleus.

2. A compound according to claim 1, wherein *n* is 0.

3. A compound according to claim 1, wherein *m* is 0.

4. A compound according to claim 1, wherein neither *m* nor *n* is 0.

5. A compound according to claim 2, which is trans - 4 - [N - (2′ - hydroxybenzylidene)aminomethyl]cyclohexane - 1 - carboxylic acid or the sodium salt thereof, trans - 4 - [N - (4′ - hydroxybenzylidene)aminomethyl]cyclohexane - 1 - carboxylic acid, trans - 4 - [N - (2′,3′ - dihydroxybenzylidene)-aminomethyl]cyclohexane - 1 - carboxylic acid or the sodium salt thereof, or trans - 4 - [N - (2′,4′ - dihydroxybenzylidene)aminomethyl]cyclohexane - 1 - carboxylic acid or the sodium salt thereof.

6. A compound according to claim 3, which is trans - 4 - [N - (2′ - methoxybenzylidene)aminomethyl]cyclohexane - 1 - carboxylic acid or the sodium salt thereof, trans - 4 - [N - (3′,4′ - dimethoxybenzylidene)aminomethyl]cyclohexane - 1 - carboxylic acid or the sodium salt thereof, or trans - 4-[N - (2′,3′,4′ - trimethoxybenzylidene)aminomethyl]cyclohexane - 1 - carboxylic acid or the sodium salt thereof.

7. A compound according to claim 4, which is trans - 4 - [N - (3′ - methoxy - 4′ - hydroxybenzylidene)aminomethyl]cyclohexane - 1 - carboxylic acid or the sodium salt thereof.

8. A process for preparing a compound of the general formula (I) or a salt or ester thereof as claimed in claim 1, which process comprises reacting a compound represented by the general formula (II):

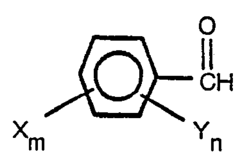

(II)

wherein X, Y, *m* and *n* are as defined in claim 1, with 4-aminomethyl-cyclohexane-1-carboxylic acid or an ester thereof and optionally either converting the resulting compound of the general formula (I) to a salt or ester thereof or converting the resulting ester of a compound of the general formula (I) to a compound of the general formula (I) or a salt or other ester thereof.

9. A pharmaceutical composition comprising a compound of the general formula (I) as claimed in claim 1 or a pharmaceutically acceptable salt or ester thereof as active ingredient, together with a pharmaceutically acceptable carrier and/or adjuvant.

10. A pharmaceutical composition according to claim 9 comprising one or more of the compounds and salts claimed in any one of claims 5 to 7.

## Revendications

1. Un composé représente par la formule générale (I):

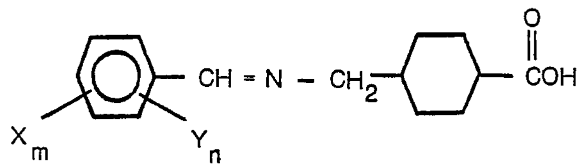

(I)

dans laquelle:

X est hydroxyle;
Y est méthoxy;
*m* est 0 ou un nombre entier compris entre 1 et 5;
*n* est 0 ou un nombre entier compris entre 1 et 5;
étant entendu que $1 \leqslant m + n \leqslant 5$,

ou un de ses sels ou esters;

étant entendu que lorsque *n* est égal à 0, *m* est égal à 2 et un des deux hydroxyles est en position para du noyau benzènique tandis que l'autre hydroxyle n'est pas en position méta du noyau benzènique.

2. Un composé selon la revendication 1, dans lequel *n* est égal à 0.

3. Un composé selon la revendication 1, dans lequel *m* est égal à 0.

4. Un composé selon la revendication 1, dans lequel ni *m* ni *n* ne sont égaux à 0.

5. Un composé selon la revendication 2, consistant en acide trans - 4 - [N - (2' - hydroxybenzylidène)aminométhyl]cyclohexane - 1 - carboxylique ou en son sel de sodium, en acide trans - 4 - [N-(4' - hydroxybenzylidène)aminométhyl]cyclohexane - 1 - carboxylique, en acide trans - 4 - [N - (2',3'-dihydroxybenzylidène)aminométhyl]cyclohexane - 1 - carboxylique ou en son sel de sodium, ou en acide trans - 4 - [N - (2',4' - dihydroxybenzylidène)aminométhyl]cyclohexane - 1 - carboxylique ou en son sel de sodium.

6. Un composé selon la revendication 3, consistant en acide trans - 4 - [N - (2' - méthoxybenzylidène)aminométhyl]cyclohexane - 1 - carboxylique ou en son sel de sodium, acide trans - 4 - [N-(3',4' - dimémethoxybenzylidène)aminométhyl]cyclohexane - 1 - carboxylique ou en son sel de sodium, ou en acide trans - 4 - [N - (2',3',4' - triméthoxybenzylidène)aminométhyl]cyclohexane - 1 - carboxylique ou en son sel de sodium.

7. Un composé selon la revendication 4, consistant en acide trans - 4 - [N - (3' - méthoxy - 4'-hydroxybenzylidene)aminométhyl]cyclohexane - 1 - carboxylique ou en son sel de sodium.

8. Procédé de préparation d'un composé de formule générale (I), ou un de ses sels ou esters, ainsi que revendiqué dans la revendication 1, ce procédé consistant à faire réagir un composé présentant la formule générale (II):

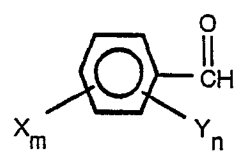

(II)

dans laquelle:

X, Y, *m* et *n* a sont tels que définis dans la revendication 1, avec un acide 4-aminométhyl-cyclohexane-1-carboxylique ou un de ses esters et éventuellement soit à convertir le composé résultant de formule générale (I) en un de ses sels ou esters, soit à convertir l'ester résultant du composé de formule générale (I) en un composé de formule générale (I) ou un de ses sels ou autre ester.

9. Composition pharmaceutique comprenant un composé de formule générale (I) ainsi que revendiqué dans la revendication 1, ou un de ses esters ou sels pharmaceutiquement acceptables en tant qu'ingrédient actif, associé à un support et/ou adjuvant acceptable du point de vue pharmaceutique.

10. Composition pharmaceutique selon la revendication 9, comprenant un ou plusieurs des composés ou de leurs sels tels que revendiqués dans une quelconque des revendications 5 à 7.

## Patentansprüche

1. Verbindung der allgemeinen Formel (I):

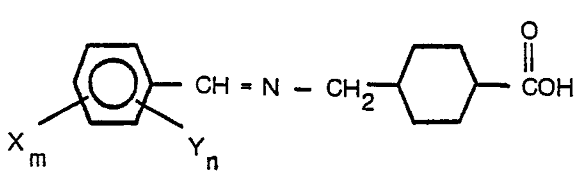

(I)

worin X eine Hydroxyl- und Y eie Methoxygruppe bedeutet und $m$ für die Zahl 0 oder eine ganze Zahl von 1 bis 5 und $n$ für die Zahl 0 oder eine ganze Zahl von 1 bis 5, unter der Bedingung, daß $1 \leqslant m + n \leqslant 5$ gilt, steht, oder ein Salz oder ein Ester der vorgenannten Verbindung, mit der Maßgabe, daß wenn $n = 0$ und $m = 2$ ist und eine der beiden Hydroxylgruppen sich in para-Stellung des Benzolkerns befindet dann die andere Hydroxylgruppe keine meta-Stellung im Benzolkern einnimmt.

2. Verbindung nach Anspruch 1, dadurch gekennzeichnet, daß $n = 0$.

3. Verbindung nach Anspruch 1, dadurch gekennzeichnet, daß $m = 0$.

4. Verbindung nach Anspruch1, dadurch gekennzeichnet, daß weder $m$ noch $n = 0$.

5. Verbindung nach Anspruch 2 entsprechend der nachfolgenden Nomenklatur: trans - 4 - [N(2′-Hydroxybenzyliden)aminomethyl]cyclohexan - 1 - karbonsäure oder das Natriumsalz hiervon, trans-4 - [N - (4′ - Hydroxybenzyliden)aminomethyl]cyclohexan - 1 - karbonsäure, trans - 4 - [N - (2′,3′-Dihydroxybenzyliden)aminomethyl] - cyclohexan - - karbonsäure oder das Natriumsalz hiervon, trans-4 - [N - (2′,4′ - Dihydroxybenzyliden)aminomethyl] - cyclohexan - 1 - karbonsäure oder das Natrium hiervon.

6. Verbindung nach Anspruch 3 entsprechend der nachfolgenden Nomenklatur: trans - 4 - [N - (2′ - Methoxybenzyliden)aminomethyl]cyclohexan - 1 - karbonsäure oder das Natriumsalz hiervon, trans - 4 - [N - (3′,4′ - Dimethoxybenzyliden)aminomethyl]cyclohexan - 1 - karbonsäure oder das Natriumsalz hiervon, trans - 4 - [N - (2′,3′,4′ - Trimethoxybenzyliden)aminomethyl]-cyclohexan - 1 - karbonsäure oder das Natriumsalz hiervon.

7. Verbindung nach Anspruch 4 entsprechend der nachfolgenden Nomenklatur: trans - 4 - [N-(3′ - Methoxy - 4′ - Hydroxybenzyliden)aminomethyl] - cyclohexan - 1 - karbonsäure oder das Natriumsalz hiervon.

8. Verfahren zur Herstellung einer Verbindung gemäß der allgemeinen Formel (I) des Anspruches 1 oder eines Salzes oder Esters hiervon, dadurch gekennzeichnet, daß eine Verbindung der allgemeinen Formel (II):

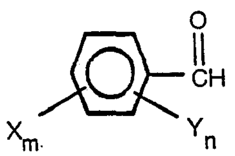

(II)

worin X, Y, $m$ und $n$ die im Anspruch 1 angegebene Bedeutung haben, mit einer 4-Aminomethyl-cyclohexan-1-karbonsäure oder einem Ester hiervon umgesetzt wird, wobei gegebenenfalls entweder die der allgemeinen Formel (I) entsprechende Verbindung in ein Salz oder einen Ester oder der von der Verbindung entsprechend der allgemeinen Formel (I) erhaltene Ester in eine Verbindung der allgemeinen Formel (I) oder in ein Salz oder einen anderen Ester umgewandelt wird.

9. Arzneimittel gekennzeichnet durch den Gehalt an einer Verbindung der allgemeinen Formel (I) nach Anspruch 1 oder einem pharmazeutisch tragbaren Salz oder Ester hiervon als Wirkstoff sowie an einem pharmazeutisch verträglichen Träger- und/oder Hilfsstoff.

10. Arzneimittel nach Anspruch 9, gekennzeichnet durch den Gehalt an einer oder mehreren der Verbindungen und Salze gemäß einem der Ansprüche 5 bis 7.

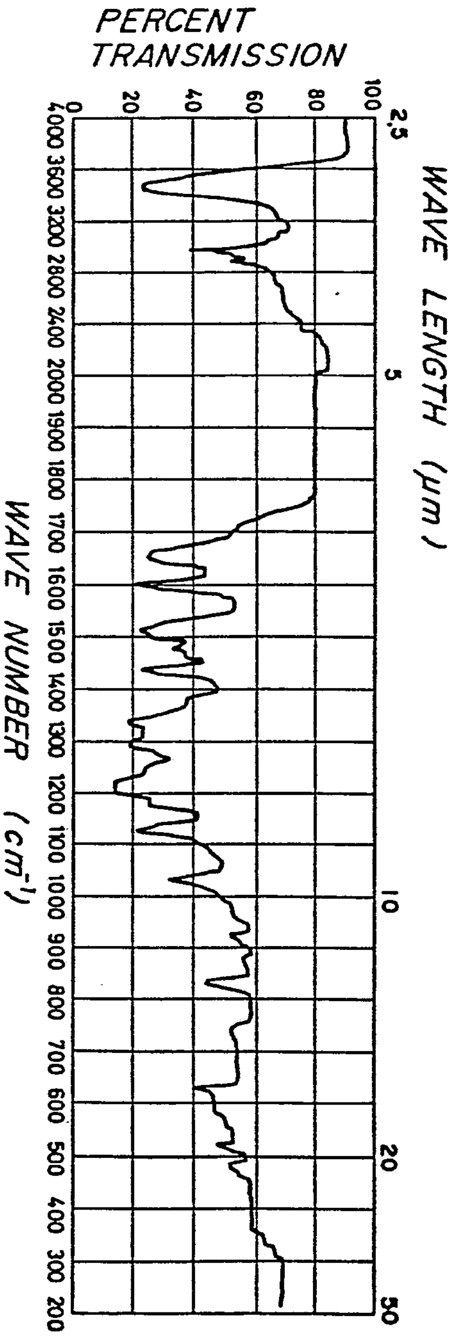
PERCENT
TRANSMISSION
0
20
40
60
80
100
WAVE LENGTH (μm)
2,5
5
10
20
50
WAVE NUMBER (cm-1)
4000 3600 3200 2800 2400 2000 1900 1800 1700 1600 1500 1400 1300 1200 1100 1000 900 800 700 600 500 400 300 200